(19) Europäisches Patentamt / European Patent Office / Office européen des brevets

(11) **EP 4 155 412 A1**

(12) **EUROPEAN PATENT APPLICATION**
published in accordance with Art. 153(4) EPC

(43) Date of publication:
**29.03.2023  Bulletin 2023/13**

(21) Application number: **21809103.1**

(22) Date of filing: **21.05.2021**

(51) International Patent Classification (IPC):
**C12Q 1/02** (2006.01)         **C11B 9/00** (2006.01)
**G01N 21/76** (2006.01)

(52) Cooperative Patent Classification (CPC):
**C11B 9/00; C12Q 1/02; G01N 21/76**

(86) International application number:
**PCT/JP2021/019359**

(87) International publication number:
**WO 2021/235544 (25.11.2021 Gazette 2021/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **22.05.2020   JP 2020089819**

(71) Applicant: **Japan Tobacco Inc.**
**Tokyo 105-6927 (JP)**

(72) Inventors:
• **SHIMOMURA, Ippei**
  **Tokyo 130-8603 (JP)**
• **MISONO, Yosuke**
  **Tokyo 130-8603 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **METHOD FOR DETERMINING FORMULATION OF COMBINATION CONSISTING OF MULTIPLE CANDIDATE SUBSTANCES FOR SMELL, TASTE OR SOMATIC SENSATION AND REPRODUCING SMELL, TASTE OR SOMATIC SENSATION OF TARGET SUBSTANCE**

(57)    The present invention relates to a method, a program, and a system for determining a formulation of a combination consisting of multiple candidate substrates of smell, taste, or somatic sensation and reproducing a smell, taste, or somatic sensation of a target substance, and a method for producing a mixture that reproduces a smell, taste, or somatic sensation of a target substance, from a combination consisting of multiple candidate substrates of smell, taste, or somatic sensation. The method for determining a formulation of a combination consisting of multiple candidate substrates of smell, taste, or somatic sensation and reproducing a smell, taste, or somatic sensation of a target substance comprises determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other, wherein the target substance is a single compound or a mixture of multiple compounds.

EP 4 155 412 A1

**Description**

TECHNICAL FIELD

The present invention relates to a method, a program, and a system for determining a formulation of a combination consisting of multiple candidate substrates of smell, taste, or somatic sensation and reproducing a smell, taste, or somatic sensation of a target substance, and a method for producing a mixture that reproduces a smell, taste, or somatic sensation of a target substance, from a combination consisting of multiple candidate substrates of smell, taste, or somatic sensation.

BACKGROUND ART

Attempts have been made to reproduce the smell, taste, or somatic sensation of a target substance by using a substance other than the target substance. For example, in circumstances where the target substance is difficult to handle, difficult to obtain, or expensive, it is desirable if a similar smell or taste can be reproduced by using a substance other than the target substance.

Japanese Patent Laid-Open No. 2003-279459 (PTL 1) discloses a method for determining an odor recipe. The method of this literature is characterized by determining, from multiple different types of element odors, a combination of element odors that is approximate to a target odor, based on multiple odor sensor response patterns.

This method in summary determines "a combination of element odors that is approximate to a target odor" by utilizing "multiple odor sensor response patterns". PTL 1 describes the method as follows: "This method for determining an odor recipe allows the target odor to be approximated by the combination of element odors. That is, rather than searching for a concentration of each one of the element odors, the method allows the target odor to be approximated based on the presence or absence of the element odors. When an odor is formulated with many element odors, the quality of the formulated odor is significantly affected by the presence or absence of the element odors, and not significantly affected by the concentration (content) of each element odor. Thus, the target odor can be approximated by focusing only on the presence or absence of each element odor, and this also limits the search range, which facilitates determining the odor recipe" ([0012] of PTL 1).

Japanese Patent Laid-Open No. 2005-043072 (PTL 2) discloses a method for recording and reproducing an odor. The method of this literature comprises the steps of detecting a target odor and a formulated odor formulated with multiple element odors with sensor arrays, and approximating response patterns of these odors.

Japanese Patent Laid-Open No. 2008-308649 (PTL 3) discloses an odor coding system that creates and encodes a recipe for synthesizing a target odorant. The odor coding system of this literature comprises:

a molecular information database that stores molecular information parameters corresponding to molecular structure information of multiple odorants;
an element odor database that stores multivariate analysis results for multiple element odorants;
a molecular information search unit that obtains corresponding molecular information parameters from the molecular information database, based on the molecular information of the target odorant;
a multivariate analysis unit that carries out multivariate analysis of the target odorant using the molecular information obtained by the molecular information search unit to obtain a multivariate analysis result;
an element odor determination unit that obtains, from the element odor database, information on element odorants for synthesizing the target odorant, based on the multivariate analysis result of the target odorant; and
an odor code output unit that encodes the information on element odorants for output.

This system in summary comprises a multivariate analysis unit that carries out multivariate analysis of a target odorant based on the molecular information of the target odorant to obtain a multivariate analysis result; and an element odor determination unit that obtains information on element odorants for synthesizing the target odorant, based on the analysis result.

Japanese Patent Laid-Open No. 2018-109099 (PTL 4) discloses a system for constructing a fragrance composition. The system of this literature "determines a combination and a ratio of multiple part notes for expressing a desired smell, through a simulation that models an observed scene of a smell, using a sensory indicator involved in human olfaction".

International Publication WO 2018/190118 (PTL 5) discloses a method for screening a candidate compound with ambergris notes from test compounds. The method disclosed in this literature is a method for screening a candidate compound with ambergris notes from test compounds, using an olfactory receptor responsive to a fragrance with ambergris notes, the method comprising the steps of:

(i) contacting the test compounds with an olfactory receptor selected from the group consisting of OR7A17, OR7C1 and proteins containing an amino acid sequence having 80% or more identity to an amino acid sequence represented by SEQ ID NO: 2 or 4 (of PTL 5) and responsive to a fragrance with ambergris notes, and measuring responses of the olfactory receptor to the test compounds;

(ii) measuring a response of the olfactory receptor used in step (i) in the absence of the test compounds; and

(iii) comparing the measurement results of steps (i) and (ii) to select a test compound with a change in responsiveness as the candidate compound with ambergris notes.

All of the methods or systems prior to the present invention are intended to match the taste or smell of a candidate substance to that of a target substance at a single point of concentration to reproduce the taste or smell. However, the response intensity of a taste or smell receptor in the living body is determined by the concentration of a substance that gives the taste or smell in the oral cavity or olfactory mucus. The methods in the prior art fail to consider whether the smell, taste, or somatic sensation of a target substance can be reproduced even if the concentrations of the target substance and/or candidate substances change.

CITATION LIST

PATENT LITERATURE

PTL 1: Japanese Patent Laid-Open No. 2003-279459
PTL 2: Japanese Patent Laid-Open No. 2005-043072
PTL 3: Japanese Patent Laid-Open No. 2008-308649
PTL 4: Japanese Patent Laid-Open No. 2018-109099
PTL 5: International Publication WO 2018/190118

NON PATENT LITERATURE

NPL 1: Joel D. Mainland, et al., Nat Neurosci. 2014; 17(1): p.114-120
NPL 2: Howard GJ et al., J. Theor. Biol. 2009 Aug 7; 259 (3): 469-477
NPL 3: Saito H, et al., Sci Signal, 2009; 2(60): ra9.
NPL 4: Joel D. Mainland, et al., 2015; Scientific Data, 2: 150002
NPL 5: Amrita Samanta, et al., Subcell Biochem. 2018; 87: 141-165

SUMMARY OF INVENTION

TECHNICAL PROBLEM

To solve the aforementioned problem, the present inventors have found that, in order to reproduce a smell, taste, or somatic sensation of a target substance even if a certain change occurs in the concentrations of the target substance and candidate substances, it is important to design a combination of candidate substances whose dose response curve is approximate to a dose response curve of the target substance, thus arriving at the present invention. Specifically, the smell, taste, or somatic sensation of the target substance can be reproduced by approximating "the relationship between the concentration of the target substance that gives the smell, taste, or somatic sensation of the target substance and the response intensity of a receptor (dose response curve of the target substance)" and "the relationship between the concentration of the combination of the candidate substances and the response intensity of a receptor (dose response curve of the combination of the candidate substances)", by adjusting the mixture ratio of each of the candidate substances.

It is an object of the present invention to provide a method, a program, and a system for determining a formulation of a combination consisting of multiple candidate substrates of smell, taste, or somatic sensation and reproducing a smell, taste, or somatic sensation of a target substance, and a method for producing a mixture that reproduces a smell, taste, or somatic sensation of a target substance, from a combination consisting of multiple candidate substrates of smell, taste, or somatic sensation.

SOLUTION TO PROBLEM

The present invention includes, without limitation, the following embodiments:

[Embodiment 1]

A method for determining a formulation of a combination consisting of multiple candidate substrates of smell, taste, or somatic sensation and reproducing a smell, taste, or somatic sensation of a target substance, the method comprising:

determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other,
wherein the target substance is a single compound or a mixture of multiple compounds.

[Embodiment 2]

A method for determining a formulation of a combination consisting of multiple candidate substrates of smell, taste, or somatic sensation and reproducing a smell, taste, or somatic sensation of a target substance, the method comprising:

(i) step 1 of setting, with respect to one or multiple desired olfactory, taste, or somatosensory receptors, a function $f_iT$ representing a response intensity of the receptor for the target substance;
(ii) step 2 of setting a function $f_iR$ representing a response intensity of the receptor for the combination consisting of the multiple candidate substrates of smell, taste, or somatic sensation and characterized by a concentration of each of the candidate substances;
(iii) step 3 of setting an error function g characterizing an error between the function $f_iT$ and the function $f_iR$ for each receptor, wherein the error function is minimized when the function $f_iT$ and the function $f_iR$ match each other;
(iv) step 4 of setting a functional F having as arguments all of error functions g; related to each receptor obtained in step 3, wherein the functional F is minimized when the error functions are minimized for all of the receptors; and
(v) step 5 of performing optimization for the functional F, and determining a concentration of each of the candidate substances when the functional F takes an optimal value as a concentration that reproduces the target smell, taste, or somatic sensation,

wherein the target substance is a single compound or a mixture of multiple compounds.

[Embodiment 3]

The method according to embodiment 1 or 2, wherein the one or multiple olfactory, taste, or somatosensory receptors include a receptor for which the target substance is a ligand.

[Embodiment 4]

The method according to embodiment 1 or 2, wherein the one or multiple olfactory, taste, or somatosensory receptors include all of receptors for which the target substance is a ligand.

[Embodiment 5]

The method according to any one of embodiments 1 to 4, wherein the one or multiple olfactory, taste, or somatosensory receptors include a receptor for which at least one of the smell, taste, or somatosensory candidate substances is a ligand.

[Embodiment 6]

The method according to any one of embodiments 1 to 5, wherein at least one of the multiple candidate substrates of smell, taste, or somatic sensation is a ligand for a receptor for which the target substance is a ligand.

[Embodiment 7]

The method according to any one of embodiments 1 to 6, wherein the receptors are multiple olfactory, taste, or somatosensory receptors.

[Embodiment 8]

The method according to any one of embodiments 2 to 7, wherein the function $f_iT$ includes as parameters a maximum response intensity $\alpha$ and an EC50 (a concentration representing 50% of a maximum response from a minimal value) of the target substance for the one or multiple olfactory, taste, or somatosensory receptors of interest.

[Embodiment 9]

The method according to any one of embodiments 2 to 8, wherein the function $f_iR$ includes as parameters a maximum response intensity $\alpha$ and an EC50 of the combination of the multiple candidate substances for the one or multiple olfactory, taste, or somatosensory receptors of interest.

[Embodiment 10]

The method according to any one of embodiments 2 to 9, further comprising:
(vi) step 6 of adjusting the concentration that reproduces the smell, taste, or somatic sensation obtained in step 5 based on a human sensory test to provide a final concentration that reproduces the smell, taste, or somatic sensation.

[Embodiment 11]

A method for producing a mixture that reproduces a smell, taste, or somatic sensation of a target substance, from a combination consisting of multiple candidate substrates of smell, taste, or somatic sensation, the method comprising:

(i) step 1 of setting, with respect to one or multiple desired olfactory, taste, or somatosensory receptors, a function $f_iT$ representing a response intensity of the receptor for the target substance;
(ii) step 2 of setting a function $f_iR$ representing a response intensity of the receptor for the combination consisting of the multiple candidate substrates of smell, taste, or somatic sensation and characterized by a concentration of each of the candidate substances;
(iii) step 3 of setting an error function g characterizing an error between the function $f_iT$ and the function $f_iR$ for each receptor, wherein the error function is minimized when the function $f_iT$ and the function $f_iR$ match each other;
(iv) step 4 of setting a functional F having as arguments all of error functions g; related to each receptor obtained in step 3, wherein the functional F is minimized when the error functions are minimized for all of the receptors;
(v) step 5 of performing optimization for the functional F, and determining a concentration of each of the candidate substances when the functional F takes an optimal value as a concentration that reproduces the target smell, taste, or somatic sensation, and
(vi) step 6 of mixing the candidate substances at the concentrations determined in step 5 to produce a mixture,

wherein the target substance is a single compound or a mixture of multiple compounds.

[Embodiment 12]

A program for determining a formulation of a combination consisting of multiple candidate substrates of smell, taste, or somatic sensation and reproducing a smell, taste, or somatic sensation of a target substance, the program causing a computer to implement the step of:

determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other,
wherein the target substance is a single compound or a mixture of multiple compounds.

[Embodiment 13]

A program for determining a formulation of a combination consisting of multiple candidate substrates of smell, taste, or somatic sensation and reproducing a smell, taste, or somatic sensation of a target substance, the program causing a computer to implement the steps of:

(i) setting, with respect to one or multiple desired olfactory, taste, or somatosensory receptors, a function $f_iT$ representing a response intensity of the receptor for the target substance;

(ii) setting a function $f_iR$ representing a response intensity of the receptor for the combination consisting of the multiple candidate substrates of smell, taste, or somatic sensation and characterized by a concentration of each of the candidate substances; and

(iii) performing optimization for a functional F, and determining a concentration of each of the candidate substances when the functional F takes an optimal value as a concentration that reproduces the target smell, taste, or somatic sensation, wherein the functional F has as arguments all of error functions $g_i$ related to each receptor, and is minimized when the error functions are minimized for all of the receptors, and wherein an error function $g_i$ related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

[Embodiment 14]

A system for determining a formulation of a combination consisting of multiple candidate substrates of smell, taste, or somatic sensation and reproducing a smell, taste, or somatic sensation of a target substance, the system being configured to:

determine a mixture ratio of the multiple candidate substrates of smell, taste, or somatic sensation such that with respect to one or multiple desired olfactory, taste, or somatosensory receptors, a dose response curve of the receptor for the substance of interest and a dose response curve of the receptor for the combination consisting of the multiple candidate substrates of smell, taste, or somatic sensation approximate each other,

wherein the substance of interest is a single compound or a mixture of multiple compounds.

[Embodiment 15]

A system for producing a mixture that reproduces a smell, taste, or somatic sensation of a target substance, from a combination consisting of multiple candidate substrates of smell, taste, or somatic sensation, the system being configured to:

(i) set, with respect to one or multiple desired olfactory, taste, or somatosensory receptors, a function $f_iT$ representing a response intensity of the receptor for the substance of interest;

(ii) set a function $f_iR$ representing a response intensity of the receptor for the combination consisting of the multiple candidate substrates of smell, taste, or somatic sensation and having a concentration of each of the candidate substances as a variable; and

(iii) perform optimization for a functional F, and determine a concentration of each of the candidate substances when the functional F takes an optimal value as a concentration of a recipe for the target smell, taste, or somatic sensation, wherein the functional F has as arguments all of error functions $g_i$ related to each receptor, and is minimized when the error functions are minimized for all of the receptors, and wherein an error function $g_i$ related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

It is noted that reproducing "a smell, taste, or somatic sensation" may include reproducing only one of the smell, taste, and somatic sensation. That is, "determining a formulation of a combination consisting of multiple candidate substrates of smell, taste, or somatic sensation" may include determining a formulation of a combination consisting of multiple candidate substances, for only one selected from the smell, taste, and somatic sensation.

Reproducing "a smell, taste, or somatic sensation" may also include reproducing at least two selected from the smell, taste, and somatic sensation. That is, "determining a formulation of a combination consisting of multiple candidate substrates of smell, taste, or somatic sensation" may include determining a formulation of a combination consisting of multiple candidate substances, for at least two selected from the smell, taste, and somatic sensation.

Reproducing "a smell, taste, or somatic sensation" may further include reproducing all of the smell, taste, and somatic sensation. That is, "determining a formulation of a combination consisting of multiple candidate substrates of smell, taste, or somatic sensation" may include determining a formulation of a combination consisting of multiple candidate substances, for all of the smell, taste, and somatic sensation.

ADVANTAGEOUS EFFECTS OF INVENTION

The present invention makes it possible to determine a suitable formulation of a combination consisting of multiple candidate substrates of smell, taste, or somatic sensation and reproducing a smell, taste, or somatic sensation of a target substance. The method of the present invention utilizes a dose response curve of the target substance and a dose response curve of the combination of the candidate substances, and thereby can reproduce the smell, taste, or somatic sensation of the target substance even if the concentrations of the target substance and candidate substances change to a certain degree.

BRIEF DESCRIPTION OF DRAWINGS

Fig. 1 shows a dose response curve model obtained for a mixture of agonists A and B with different EC50s and maximum response intensities.
Fig. 2 shows predicted dose response curves of a coumarin reconstitution mixture. The solid lines represent predicted dose response curves of the coumarin reconstitution mixture, and the dotted lines represent dose response curves of 1 M coumarin.
Fig. 3 shows dose response curves of the coumarin reconstitution mixture. The triangles represent dose response curves of the coumarin reconstitution mixture, and the circles represent dose response curves of 1 M coumarin.
Fig. 4 shows the results of evaluating the similarity in odor quality between the coumarin reconstruction fragrance and coumarin using a VAS.
Fig. 5 shows one example of a hardware configuration of a computer.
Fig. 6 shows predicted dose response curves and dose response curves of a lavender oil reconstitution mixture. The dotted lines represent predicted dose response curves of the lavender oil reconstitution mixture, the triangles (solid lines) represent dose response curves of the lavender oil reconstitution mixture, and the circles (solid lines) represent dose response curves of lavender oil.
Fig. 7 shows the results of sensory evaluation for evaluating the similarity in odor quality between the lavender oil reconstitution fragrance and lavender oil using a VAS.

DESCRIPTION OF EMBODIMENTS

The present inventors have found that a smell, taste, or somatic sensation of a target substance can be reproduced by approximating "the relationship between the concentration of the target substance that gives the smell, taste, or somatic sensation of the target substance and the response intensity of a receptor (dose response curve of the target substance)" and "the relationship between the concentration of the combination of the candidate substances and the response intensity of a receptor (dose response curve of the combination of the candidate substances)", by adjusting the mixture ratio of each of the candidate substances. One embodiment for specifically carrying out this will be hereinafter described. Without limitation, the present invention includes the following.

I. Method for determining a formulation of a combination consisting of multiple candidate substrates of smell, taste, or somatic sensation and reproducing a smell, taste, or somatic sensation of a target substance

The present invention relates to a method for determining a formulation of a combination consisting of multiple candidate substrates of smell, taste, or somatic sensation and reproducing a smell, taste, or somatic sensation of a target substance. The method of the present invention comprises:

determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other,
wherein the target substance is a single compound or a mixture of multiple compounds.

The method combines the multiple candidate substrates of smell, taste, or somatic sensation for the purpose of reproducing the smell, taste, or somatic sensation of the target substance, wherein the formulation of the combination is determined to be more suitable or the most suitable. As used herein, "reproducing" includes approximating the smell, taste, or somatic sensation of the target substance. That is, the present invention includes determining a formulation of a combination consisting of multiple candidate substrates of smell, taste, or somatic sensation by using the method of the present invention to thereby approximate the smell, taste, or somatic sensation of the target substance more closely, compared to when combining a multiple candidate substrates of smell, taste, or somatic sensation in any manner, without using the method. A determination as to whether the smell, taste, or somatic sensation of the target substance has been reproduced can be made, for example, through sensory evaluation by a panel of trained experts.

"Target substance" is not specifically limited as long as it is a substance that causes a living body to perceive the smell, taste, or somatic sensation, and may be a natural or artificial substance. Examples include, without limitation, smell substances, such as coumarin, vanillin, limonene, and lavender oil. Coumarin is a type of aroma component of plants, represented by cherry leaves. Lavender oil is an essential oil obtained from plants of the lavender genus (*Lavendula*) of the Perilla family, and is used as a fragrance. Examples also include taste substances, such as caffeine (bitter taste), saccharin (bitter and sweet tastes), and glutamic acid (umami taste). Examples also include substances that give somatic sensations, such as allyl isothiocyanate (pain sensation), capsaicin (warm sensation), and menthol (cold sensation).

As described above, the target substance may be a single compound or a mixture of multiple compounds. When the target substance is a single compound, it is desirable to employ, without limitation, molar concentration (M or mol/L) as the unit of concentration of the target substance. Alternatively, when the target substance is a mixture of multiple compounds, it is desirable to employ the dilution ratio relative to any specified concentration taken as 1, for the concentration of each of the candidate substances in the mixture or the concentration of the mixture itself of the candidate substances. When the concentration of an undiluted stock solution of the mixture, i.e., the concentration at a dilution ratio of 1, is hypothetically taken as the concentration corresponding to 1 mol/L of a single compound, the concentration can be expressed on an axis of concentration equivalent to the molar concentration in a simulated manner. As used herein, the any specified concentration may be the concentration of the stock solution of the mixture purchased, extracted, prepared, or otherwise obtained, or may be the value as diluted or concentrated appropriately based on a specific indicator.

"Smell, taste, or somatosensory candidate substances" are also not specifically limited in type, and may be natural or artificial substances. The method uses a combination consisting of multiple candidate substrates of smell, taste, or somatic sensation. In the examples of the present specification, a smell approximate to coumarin was reproduced by determining a suitable formulation of a combination of three candidate substances, i.e., piperonal, $\gamma$-heptalactone, and menthone. "Smell, taste, or somatosensory candidate substances" may be substances that cause a living body to perceive the smells, tastes, or somatic sensations, or may be substances that when combined with another substance inhibit the smell, taste, or somatic sensation of the other substance. "Smell, taste, or somatosensory candidate substances" may be referred to herein as "candidate substances".

The "candidate substances" are not specifically limited as described above; however, in one embodiment, the candidate substances may be selected in advance by screening or the like, prior to being subjected to the method for determining a formulation of a combination of candidate substances. As the smell candidate substances, substances with a smell similar to that of the target substance may be selected based on smell characteristics obtained from sensory evaluation or publicly available data of known fragrances, for example. As the taste candidate substances, substances with a taste similar to that of the target substance may be selected based on taste characteristics obtained from sensory evaluation or publicly available data of known food additives, for example. As the somatosensory candidate substances, substances with a somatic sensation similar to that given by the target substance may be selected based on somatosensory characteristics obtained from sensory evaluation or publicly available data of known substances that give somatic sensations, for example.

"Formulation" means a combination consisting of multiple candidate substrates of smell, taste, or somatic sensation and a mixture ratio thereof. When the candidate substances are a mixture of multiple compounds, it is desirable to employ, without limitation, the dilution ratio relative to any specified concentration taken as 1, for the concentrations of the candidate substances or the concentration of the mixture itself of the candidate substances. When the concentration of an undiluted stock solution of the mixture, i.e., the concentration at a dilution ratio of 1, is hypothetically taken as the concentration corresponding to 1 mol/L of a single compound, the concentration can be expressed on an axis of concentration equivalent to the molar concentration in a simulated manner. As used herein, the any specified concentration may be the concentration of the stock solution of the mixture purchased, extracted, prepared, or otherwise obtained, or may be the value as diluted or concentrated appropriately based on a specific indicator. The mixture ratio of each of the candidate substances in the mixture of the multiple candidate substrates of smell, taste, or somatic sensation means a relative mixture ratio of the concentration of each candidate substance.

The method includes comparing, with respect to "one or multiple desired olfactory, taste, or somatosensory receptors", a dose response curve of the receptor for the target substance and a dose response curve of the receptor for the combination of the candidate substances, and determining a mixture ratio of each of the candidate substances in the combination of the candidate substances such that these dose response curves approximate each other.

Olfactory receptors are G protein-coupled receptors, as with taste receptors, such as bitter taste receptors, sweet taste receptors, and umami taste receptors. Olfactory receptors are present on olfactory cells of the olfactory epithelium. Olfactory cells are the only sites in the human body where the neurons are in direct contact with the external world, and have the other end thereof directly connected to the area of the brain called the olfactory bulb. "Odorants" are low-molecular-weight compounds with a molecular weight of about 30 to 300, and hundreds of thousands of odorants are said to be present on the earth. About 400 functional olfactory receptors have been identified in humans, and are grouped into 18 gene families, including pseudogenes. Each receptor responds to many similar structures, rather than responding to a single odorant. Moreover, many odorants stimulate more than one receptor. While ligands corresponding to about 100 olfactory receptors have been identified in the past, little has been revealed about an odor exhibited by each receptor, except in a few cases, such as PTL 5.

Binding of an odorant to an olfactory receptor causes an increase in intracellular calcium concentration through a signal transduction process mediated by intracellular second messengers and the like, as with taste receptors, such as bitter taste receptors, sweet taste receptors, and umami taste receptors. A detailed description of olfactory receptors can be found in reviews, such as "Kagaku Juyouno Kagaku: Nioi Aji Feromon Bunshi Kara Koudou Made" (in Japanese) ("Chemosensory sciences: odor, taste and pheromones, from molecules to behaviors") (Kazunari Higashihara (ed.), Kagaku-Dojin, 2012).

G protein-coupled receptors are a type of receptors present on the constitutional membrane within the cell or on the cytoplasmic membrane of eukaryotes. Upon receiving various signals (such as neurotransmitters, hormones, chemical substances, and light) from outside the cell (outside the membrane), G protein-coupled receptors undergo conformational changes, which activate trimeric G proteins bound to the inside of the membrane, leading to signal transduction. G proteins are heterotrimers that are bound to the inner surface of the cell membrane and in which the G$\beta\gamma$ dimer is tightly bound to the G$\alpha$ subunit. When ligands bind to G protein-coupled receptors for activation, downstream effector proteins (for example, G proteins) are activated. G protein-coupled receptors and downstream G proteins regulate many fundamental physiochemical reactions in intracellular signaling networks, such as olfaction, gustation, vision, neurotransmission, metabolism, cell differentiation and proliferation, inflammatory reactions and immune responses.

Gustation is the sensation produced when a substance is taken into the mouth, by binding of the substance to a specific receptor present particularly on the tongue surface. Mammalian gustation is made up of five basic tastes, i.e., salty taste, sour taste, sweet taste, umami taste, and bitter taste, and is believed to be constituted by integration of these basic tastes. At present, salty taste and sour taste are said to be detected via several ionotropic receptors expressed on the cell membrane on the proximal side of taste cells present in the taste buds on the tongue surface.

Sweet taste, umami taste, and bitter taste are believed to be detected by signal transduction mediated by G protein-coupled receptors present on taste cells and G proteins that couple thereto. Specifically, it has been revealed that bitter taste is received by molecules named T2R family (bitter taste receptors) (25 types in humans); sweet taste is received by a heterodimer of T1R2 + T1R3 (sweet taste receptor); and umami taste is received by a heterodimer of T1R1 + T1R3 (umami taste receptor).

The transduction mechanism of taste information is generally understood to be as follows: First, binding of a taste substance to a receptor on taste cells causes an increase in intracellular calcium concentration through a signal transduction process mediated by intracellular second messengers (IP3 and DAG) and the like. A calcium ion supplied into a cell then releases a neurotransmitter into a synapse to generate an action potential in the neuron and, as a result, a taste signal initiated from the receptor is transduced from the taste neuron to the brain, where the taste information is identified and determined. A detailed description of taste receptors can be found in reviews, such as "Kagaku Juyouno Kagaku: Nioi Aji Feromon Bunshi Kara Koudou Made" (in Japanese) ("Chemosensory sciences: odor, taste and pheromones, from molecules to behaviors") (Kazunari Higashihara (ed.), Kagaku-Dojin, 2012).

The term "somatosensory" or "somatic sensation" is generally used as the collective term for sensations such as tactile, temperature, pain, and deep sensations induced by a temperature stimulus, a chemical stimulus, and a mechanical stimulus. As used herein, this term means somatic sensations elicited through chemical stimuli, as with gustation and olfaction.

TRP channels are representative receptors involved in somatic sensations in response to chemical stimuli. TRP channels are a type of non-selective cation channels, and 28 types of TRP channels made up of six subfamilies have been reported in mammals. Among them, TRPA1, TRPM8, and TRPV1, which are strongly expressed on sensory nerves, are known to elicit somatic sensations, such as pain and temperature sensations in response to exogenous chemical stimuli. TRPA1 is a receptor mainly involved in pain sensation in humans, and is activated by allyl isothiocyanate, cinnamaldehyde, or the like contained in wasabi or mustard. TRPM8 is a receptor involved in cold sensation, and is activated by a temperature stimulus at 28°C or less or a chemical stimulus with a cooling agent, such as menthol. TRPV1 is a receptor involved in pain and warm sensations, and is activated by a temperature stimulus at 42°C or more or capsaicin. Each TRP channel when activated causes the influx of cations into cells, which depolarizes the neurons, leading to transduction of sensory information. For TRP channels, reference may be made to, for example, Amrita Samanta, et al., Subcell Biochem. 2018; 87: 141-165.

In the method for determining a formulation of a combination consisting of multiple candidate substrates of smell, taste, or somatic sensation and reproducing a smell, taste, or somatic sensation of a target substance, the receptors of "olfactory, taste, or somatosensory receptors" are not specifically limited in type. In one embodiment, the receptors are preferably human olfactory, taste, or somatosensory receptors. The number of the receptors may be one, or more than one.

"Ligand" generally refers to a substance that specifically binds to a specific receptor. The ligand generally means a single compound. On the other hand, in research on olfaction, gustation, or somatic sensations, evaluating not only a single compound but also a mixture of multiple substances as substances that give a smell, taste, or somatic sensation is of great significance. The mixture includes, for example, a mixture such as a prepared fragrance or an extract with a smell such as an essential oil related to olfaction; and an extract from a processed food or a fresh food related to gustation. In the method for determining a formulation of a combination consisting of multiple candidate substrates of smell, taste, or somatic sensation and reproducing a smell, taste, or somatic sensation of a target substance, the target substance includes not only a single compound but also a mixture containing multiple ligands (pure substances or single compounds) that selectively and specifically bind to the receptors.

Chemical substances contained in foods, beverages, and the like, neurotransmitters, hormones, and the like have the possibility of serving as G protein-coupled receptor agonists in a series of signal transduction events, in which they bind to G protein-coupled receptors as ligands, which causes conformational changes in the receptors, which activate G proteins bound to the inside of the membrane. The ligand is preferably an agonist, which is a substance that acts on a receptor in a living body and has the same possibility as a neurotransmitter, a hormone, or the like.

A taste or a smell is generally recognized as an integration of information from multiple receptors. For example, the fragrances L-carvone and coumarin share multiple identical receptors that respond thereto.

[Table 1]

| Compound | OR2W1 | OR5P3 | OR1A1 | OR8B3 | OR1C1 | OR2B11 | OR2J2 |
|---|---|---|---|---|---|---|---|
| (-)-Carvone | | | | | | | |
| Coumarin | | | | | | | |

Low ◁▷ High

EC50

(See Saito H, et al., Sci Signal, 2009; 2(60): ra9. and Joel D. Mainland, et al., 2015; Scientific Data, 2: 150002.)

On the other hand, the smells of L-carvone and coumarin are spearmint-like and *sakura mochi*-like, respectively, and it is difficult for a person other than an expert to find a similarity between L-carvone and coumarin. Presumably, this is because even if the same receptors respond, the degree of response that depends on concentration has a significant effect on the smell. The presence of a receptor that strongly responds to one but not to the other is also believed to have an effect. In the method for determining a formulation of a combination consisting of multiple candidate substrates of smell, taste, or somatic sensation and reproducing a smell, taste, or somatic sensation of a target substance, the olfactory, taste, or somatosensory receptors are multiple olfactory, taste, or somatosensory receptors, without limitation. In one embodiment, the receptors desirably include more types of receptors. The receptors include, as a desirable receptor, a receptor for which the target substance is a ligand, more preferably include more receptors for which the target substance is a ligand, and still more preferably include all of receptors for which the target substance is a ligand.

In one embodiment, the one or multiple olfactory, taste, or somatosensory receptors include a receptor for which at least one of the smell, taste, or somatosensory candidate substances is a ligand. In one embodiment, at least one of the multiple candidate substrates of smell, taste, or somatic sensation is a ligand for a receptor for which the target substance is a ligand.

The dose response curve means a curve with respect to a receptor of interest, plotting the concentration of the substance (including a compound and a mixture) administered against the response intensity of the receptor for the substance. For any dose response curve of an olfactory, taste, or somatosensory receptor, a model having the concentration of the substance as a variable and having a maximum response intensity $\alpha$ and an EC50 (a concentration representing 50% of a maximum response from a minimal value) of the substance as parameters can be preferably used to depict the dose response curve. The concentration of the substance is preferably expressed using a logarithm, in which case the dose response curve is a sigmoid curve (log-sigmoid curve). It is noted that the base of the logarithm used in this case may be 10, e, or anything else.

In the method for determining a formulation of a combination consisting of multiple candidate substrates of smell, taste, or somatic sensation and reproducing a smell, taste, or somatic sensation of a target substance, a mixture ratio of the multiple candidate substrates of smell, taste, or somatic sensation is determined such that a dose response curve of the receptor for the target substance and a dose response curve of the receptor for the combination of the candidate substances "approximate each other". The response intensity of a taste, smell, or somatosensory receptor in a living body is determined by the concentration of a substance that gives the taste, smell, or somatic sensation in the oral cavity or olfactory mucus. On the other hand, it is difficult to measure the amount and concentration of such a substance, and the amount and concentration of the substance easily vary depending on the amount of a food ingested, the manner of smelling, the amount of inhalation, and the like. When the response for the target substance and the response for the combination of the candidate substances are matched at only a single point of concentration, there would be no difference in response intensity of the olfactory receptor if these dose response curves happen to be equal to each other. However, if there is a large difference in EC50, a certain difference in concentration may produce a significant difference in response, which may lead to a difference in smell or taste. It is therefore important to design a combination of candidate substances having an approximate dose response curve, which is not affected by a certain difference in concentration.

"Approximate each other" can mean that, for example, when the function representing the dose response curve of the receptor for the target substance is designated as fT, and the function representing the dose response curve of the receptor for the combination of the candidate substances is designated as fR, and when the distance between the function fT and the function fR is defined appropriately based on the difference between the values of the function fT and the function fR at the same concentration, the distance is adjusted to fall in a predetermined range. It is noted that, for example, the distance between the function fT and the function fR can be defined as the square, the sum or the average value of absolute values of the difference between the values of the function fT and the function fR at each concentration.

In one embodiment, a method for determining a formulation of a combination consisting of multiple candidate substrates of smell, taste, or somatic sensation and reproducing a smell, taste, or somatic sensation of a target substance comprises the following steps:

(i) step 1 of setting, with respect to one or multiple desired olfactory, taste, or somatosensory receptors, a function $f_iT$ representing a response intensity of the receptor for the target substance ("i" is a subscript for identifying the corresponding receptor; $1 \leq i \leq n$; n is the total number of the receptors to be considered; the same applies below);
(ii) step 2 of setting a function $f_iR$ representing a response intensity of the receptor for the combination consisting of the multiple candidate substrates of smell, taste, or somatic sensation and characterized by a concentration of each of the candidate substances;
(iii) step 3 of setting an error function $g_i$ characterizing an error between the function $f_iT$ and the function $f_iR$ for each receptor, wherein the error function is minimized when the function $f_iT$ and the function $f_iR$ match each other;
(iv) step 4 of setting a functional F having as arguments all of error functions $g_i$ related to each receptor obtained in step 3, wherein the functional F is minimized when the error functions are minimized for all of the receptors; and
(v) step 5 of performing optimization for the functional F, and determining a concentration of each of the candidate substances when the functional F takes an optimal value as a concentration that reproduces the target smell, taste, or somatic sensation.

The function $f_iT$ may have the concentration of the target substance as an argument. The function $f_iR$ may have as an argument the concentration (hereinafter also referred to as the "dilution ratio") of the combination consisting of the multiple candidate substrates of smell, taste, or somatic sensation as a whole. This is because, as described above, by employing the dilution ratio relative to any specified concentration of the mixture taken as 1, the dose response curve can be expressed on an axis of concentration equivalent to the molar concentration in a simulated manner.

The error function $g_i$ may be a functional having as arguments the function $f_iT$ and the function $f_iR$, or may be a function having as arguments parameters characterizing the function $f_iT$ and the function $f_iR$. It is noted that the parameters characterizing the function $f_iR$ may include the concentration of each of the candidate substances.

The functional F may be substantially considered as a functional having as arguments all of the functions $f_iT$ and the functions $f_iR$ related to each receptor. Alternatively, the functional F may be substantially considered as a function having as arguments parameters characterizing all of the functions $f_iT$ and the functions $f_iR$ related to each receptor.

Based on the definition of the functional F as described above, the value of the functional F depends on the concentration of each of the candidate substances characterizing the function $f_iR$ or the function $f_iT$. The optimization for the functional F may include determining the concentration of each of the candidate substances characterizing the function $f_iR$ when the functional F takes an optimal value. Determining the concentration of each of the candidate substances characterizing the function f;R may include determining the function $f_iR$. It is noted that the optimal value of the functional F may be the minimum value or the minimal value of the functional F.

As used herein, the target substance may be a single compound or a mixture of multiple compounds.

In step 1, with respect to one or multiple desired olfactory, taste, or somatosensory receptors, a function $f_iT$ is set representing a response intensity of the receptor for the target substance and having as an argument the concentration of the target substance. The function $f_iT$ is a function representing a dose response curve of the receptor for the target substance.

For receptor i, one example of the function $f_iT$ is given as follows:

[Formula 1]

$$f_iT(c) = R_{i(T)} = \frac{\alpha_{i(T)}c}{K_{i(T)} + c} \quad (1)$$

wherein:

$R_{i(T)}$: response intensity;
c: concentration of the target substance;
$\alpha_{i(T)}$: maximum response intensity; and
$K_{i(T)}$: EC50.
$\alpha_{i(T)}$ and $K_{i(T)}$ can be determined using a known method, for example, experimentally. When these values are known, the known values may be adopted.

The function set in step 1 is not limited thereto, and may be modified appropriately; however, the function set in step 1 preferably has two parameters, and more preferably has a maximum response intensity and an EC50 as parameters.

In one embodiment, the function $f_iT$ includes as parameters a maximum response intensity $\alpha_{i(T)}$ and an EC50 (a concentration representing 50% of a maximum response from a minimal value) $K_{i(T)}$ of the target substance for the one or multiple olfactory, taste, or somatosensory receptors of interest.

When there are multiple receptors of interest, $f_iT$ is set for each of the receptors. Specifically, $f_1T$, $f_2T$ ... $f_nT$ are set for receptors 1, 2, ... n of interest.

In step 2, when a combination consisting of multiple candidate substrates of smell, taste, or somatic sensation is exposed to the receptor selected in step 1, a function f;R is set representing the relationship of the response intensity of the receptor for the combination and having as an argument the concentration, i.e., dilution ratio, of the combination of the candidate substances as a whole. This function represents the dose response curve of the receptor for the combination of multiple candidate substances. It is noted that even if the combination is diluted, the mixture ratio of the candidate substances contained therein is constant. For receptor i, one example of this function is given below:

[Formula 2]

$$f_iR(X) = R_{i(eff)} = \frac{\alpha_{i(eff)}X}{K_{i(eff)} + X} \quad (2)$$

wherein:

$$\alpha_{i(eff)} = \frac{\left(\frac{\alpha_{i1}C_1}{K_{i1}} + \frac{\alpha_{i2}C_2}{K_{i2}} + \cdots + \frac{\alpha_{im}C_m}{K_{im}}\right)}{\frac{C_1}{K_{i1}} + \frac{C_2}{K_{i2}} + \cdots + \frac{C_m}{K_{im}}}$$

$$K_{i(eff)} = \frac{1}{\frac{C_1}{K_{i1}} + \frac{C_2}{K_{i2}} + \cdots + \frac{C_m}{K_{im}}}$$

$R_{i(eff)}$: response intensity of the combination;
$\alpha_{i(eff)}$: maximum response intensity of the combination;

$K_{i(eff)}$: EC50 of the combination;

$\alpha_{ij}$: maximum response intensity of the $j^{th}$ ($1 \leq j \leq m$; m is the total number of the candidate substances) candidate substance;

$C_j$: concentration of the $j^{th}$ candidate substance in the stock solution;

$K_{ij}$: EC50 of the $j^{th}$ candidate substance; and

X: dilution ratio relative to the stock solution of the combination of the candidate substances.

The present inventors have confirmed that this model formula represents a dose response curve of a combination consisting of multiple candidate substances (see Example 3). In fact, $\alpha_{ij}$ and $K_{ij}$ of each candidate substance can be determined using a known method, for example, experimentally. When these values are known, the known values may be directly adopted as appropriate. It is noted that the value or ratio of $C_j$ is unknown in step 2, and is determined in step 5.

It is noted that the function set in step 2 is not limited to that of formula (2) above, and may be modified appropriately. When there are multiple receptors of interest, f;R is set for each of the receptors. Specifically, $f_1R$, $f_2R$ ... $f_nR$ are set for receptors 1, 2, ... n of interest.

In one embodiment, the function f;R includes as parameters a maximum response intensity $\alpha_{i(eff)}$ and an EC50$K_{i(eff)}$ of the combination of the multiple candidate substances for the one or multiple olfactory, taste, or somatosensory receptors of interest.

In step 3, an error function g; characterizing an error between the function $f_iT$ obtained in step 1 and the function f;R obtained in step 2 is set for each receptor. Here, the "error function $g_i$" is set to be minimized when $f_iT$ and f;R match each other. This is a condition for approximating "the relationship between the concentration of the target substance and the response intensity of a receptor (dose response curve of the target substance)" and "the relationship between the concentration of the combination of the candidate substances and the response intensity of a receptor (dose response curve of the combination of the candidate substances)". Without limitation, one example of this function is given below:
[Formula 3]

$$g_i\left[\kappa_{i(T)}, \alpha_{i(T)}, \kappa_{i(eff)}, \alpha_{i(eff)}\right] :=$$

$$\begin{cases} \left(\dfrac{\kappa_{i(eff)} - \kappa_{i(T)}}{\kappa_{(T)}}\right)^2 + \left(\dfrac{\alpha_{i(eff)} - \alpha_{i(T)}}{\alpha_{(T)}}\right)^2 & for\ i\ s.t.\ \alpha_{i(T)} > 0 \\ \left(\dfrac{\alpha_{i(eff)}}{\alpha_{(T)}}\right)^2 & for\ i\ s.t.\ \alpha_{i(T)} = 0 \end{cases} \quad (3)$$

$$\kappa_{i(eff)} := log\left[K_{i(eff)}\right], \kappa_{i(T)} := log\left[K_{i(T)}\right] \quad (4)$$

wherein the subscript i represents the type of receptor; T represents the target substance; and eff represents the combination of the candidate substances. $\kappa_{i(eff)}$ and $\kappa_{i(T)}$ are the common logarithm values of EC50$K_{i(eff)}$ and $K_{i(T)}$, respectively.

The above-described error function g; is set such that the values of the two curve parameters $K_{i(eff)}$ (EC50) and $\alpha_{i(eff)}$ (maximum response intensity) characterizing the dose response curve of the combination of the multiple candidate substances become smaller as they approximate the curve parameters $K_{i(T)}$ and $\alpha_{i(T)}$ related to the dose response curve of the substance of interest. While the error function is set here using the sum of the squares of the differences in each parameter, this is not limiting, and a person skilled in the art can appropriately employ the absolute value, the square root, or the like to set the error function.

In the method for determining a formulation of a combination of candidate substances, it is desirable, without limitation, to avoid the combination of the candidate substances from exhibiting a smell, taste, or somatic sensation that is not exhibited by the target substance. It is thus desirable that the dose response curve of the combination of the candidate substances also approximate that for a receptor for which the target substance is not a ligand. That is, it is desirable to adjust the dose response curve of the combination such that the response to the receptor approaches zero at least in the range of concentrations where actual use is contemplated. Examples of methods for achieving this include reducing the value of $\alpha_{i(eff)}$ for a receptor for which the target substance is not a ligand (g; is minimized when $\alpha_{i(eff)} = 0$); and increasing the value of $K_{i(eff)}$ sufficiently; wherein one or both of these methods may be performed.

In view of the above, formula (3) above separately includes the case where $\alpha_{i(T)} > 0$ for a receptor for which the target substance (fragrance) is a ligand and the case where $\alpha_{i(T)} = 0$ for a receptor for which the target substance is not a ligand, although this is not limiting.

In the error function $g_i$, $\kappa_{i(eff)}$ and $\kappa_{i(T)}$ may be the values of $K_{i(eff)}$ and $K_{i(T)}$ as they are. Preferably, from the viewpoint of aligning the scales of the difference between $K_{i(eff)}$ and $K_{i(T)}$ and the difference between $\alpha_{i(eff)}$ and $\alpha_{i(t)}$, logarithms can be used as in formula (4) above.

It is noted that in formula (3) above, normalization is performed in view of the fact that the typical values of the parameters $\kappa_{i(eff)}$, $\kappa_{i(T)}$, $\alpha_{i(eff)}$, and $\alpha_{i(T)}$ are different. That is:

[Formula 4]

$$\overline{\kappa_{(T)}} := \frac{1}{N} \sum_{i,\alpha_{i(T)}\neq 0} \kappa_{i(T)} \,, \overline{\alpha_{(T)}} := \frac{1}{N} \sum_{i,\alpha_{i(T)}\neq 0} \alpha_{i(T)}$$

wherein N may be the number of receptors for which the target substance is a ligand. Therefore:

[Formula 5]

$$\overline{\kappa_{(T)}}, \overline{\alpha_{(T)}}$$

is the average value of each parameter for the receptors that respond to the target substance. Although this is not limiting, normalization may be thus performed as appropriate, in view of the order of each parameter.

The function set in step 3 is not limited to that of formula (3), and may be modified appropriately. When there are multiple receptors of interest, g; may be set for each of the receptors. Specifically, $g_1$, $g_2$ ... $g_n$ may be set for receptors 1, 2, ... n.

Alternatively, the error function g; may be set as a model formula that minimizes the area between the curves of the two dose response curves $f_iT$ and $f_iR$.

Step 4 is a step required when there are multiple receptors of interest, and sets a functional F having as arguments all of error functions g; related to each receptor obtained in step 3. In this step, the functional F is set to be minimized when the error functions are minimized for all of the receptors. One example of the function F is given below:
[Formula 6]

$$F[g_1, g_2, \cdots, g_n] := \sum_{i=1}^{n} g_i \left[ \kappa_{i(T)}, \alpha_{i(T)}, \kappa_{i(eff)}, \alpha_{i(eff)} \right] \qquad (5)$$

This is the sum of $g_i$ exemplified in step 3 for each receptor. It is noted that since g; exemplified is a function having as arguments $\kappa_{i(T)}$, $\alpha_{i(T)}$, $\kappa_{i(eff)}$, and $\alpha_{i(eff)}$, the functional F exemplified by formula (5) can also be considered as a function having as arguments $\kappa_{i(T)}$, $\alpha_{i(T)}$, $\kappa_{i(eff)}$, and $\alpha_{i(eff)}$.

Step 5 is the step of performing optimization for the functional F obtained in step 4, and determining a concentration of each of the candidate substances when the functional F takes an optimal value as a concentration that reproduces the target smell, taste, or somatic sensation. A decrease in the value of the functional F means that the dose response curve of the target substance and the dose response curve of the combination of the multiple candidate substances approximate more closely, for each receptor. The present inventors have found that when these two dose response curves approximate each other, the smell, taste, or somatic sensation of the target substance and the smell, taste, or somatic sensation of the combination consisting of the multiple candidate substrates of smell, taste, or somatic sensation are closer to each other, leading to the reproduction of the smell, taste, or somatic sensation of the target substance. It is noted that while the optimal value may be global (minimum value) or local (minimal value), the global optimal value is preferred.

Without limitation, the optimal value of the functional F may be determined by an algebraic method when it can be solved analytically. Alternatively, the optimal value of the functional F may be determined by an optimization method using an algorithm. Examples of the latter method include, but are not limited to, gradient methods such as the steepest descent method and Newton's method; evolutionary algorithms such as genetic algorithms; estimation algorithms based on probability distribution theory such as Bayesian optimization; and randomized algorithms such as Monte Carlo methods. In the optimization process, a constraint condition on concentration may be applied using a method such as the method of Lagrange multipliers. It is noted that the optimization includes determining the parameter characterizing the function $f_iR$ when the functional F takes an optimal value, i.e., the value or ratio of $C_j$. The optimization may include determining $\kappa_{i(eff)}$ and $\alpha_{i(eff)}$ to determine the value or ratio of $C_j$.

In one embodiment, the method for determining a formulation of a combination consisting of multiple candidate substrates of smell, taste, or somatic sensation and reproducing a smell, taste, or somatic sensation of a target substance may include step 6 of adjusting the concentration that reproduces the smell, taste, or somatic sensation obtained in steps 1 to 5 based on a human sensory test to provide a final concentration that reproduces the smell, taste, or somatic sensation. For example, the concentration of each candidate substance may be adjusted by performing a sensory evaluation step by trained panelists. Through steps 1 to 5, it is possible to determine the formulation of the combination consisting of the multiple candidate substrates of smell, taste, or somatic sensation and reproducing the smell, taste, or somatic sensation of the target substance much more efficiently than by determining the mixture concentration by human sensory evaluation only. However, by making an adjustment lastly based on human sensory evaluation, it is possible to make the smell, taste, or somatic sensation even closer to the smell, taste, or somatic sensation perceived by a human.

II. Method for producing a mixture that reproduces a smell, taste, or somatic sensation of a target substance, from a combination consisting of multiple candidate substrates of smell, taste, or somatic sensation

The present invention relates to a method for producing a mixture that reproduces a smell, taste, or somatic sensation of a target substance, from a combination consisting of multiple candidate substrates of smell, taste, or somatic sensation. The method of the present invention comprises:

(i) step 1 of setting, with respect to one or multiple desired olfactory, taste, or somatosensory receptors, a function $f_iT$ representing a response intensity of the receptor for the target substance;
(ii) step 2 of setting a function $f_iR$ representing a response intensity of the receptor for the combination consisting of the multiple candidate substrates of smell, taste, or somatic sensation and characterized by a concentration of each of the candidate substances;
(iii) step 3 of setting an error function $g_i$ characterizing an error between $f_iT$ and $f_iR$ for each receptor, wherein the error function is minimized when the function $f_iT$ and the function $f_iR$ match each other;
(iv) step 4 of setting a functional F having as arguments all of error functions $g_i$ related to each receptor obtained in step 3, wherein the functional F is minimized when the error functions are minimized for all of the receptors;
(v) step 5 of performing optimization for the functional F, and determining a concentration of each of the candidate substances when the functional F takes an optimal value as a concentration that reproduces the target smell, taste, or somatic sensation, and
(vi) step 6 of mixing the candidate substances at the concentrations determined in step 5 to produce a mixture,

wherein the target substance is a single compound or a mixture of multiple compounds.

Steps 1 to 5 are as described above in "I. Method for determining a formulation of a combination consisting of multiple candidate substrates of smell, taste, or somatic sensation and reproducing a smell, taste, or somatic sensation of a target substance". The method for producing a mixture that reproduces a smell, taste, or somatic sensation of a target substance, from a combination consisting of multiple candidate substrates of smell, taste, or somatic sensation may include, after step 5, step 6 of adjusting the concentration that reproduces the smell, taste, or somatic sensation obtained in steps 1 to 5 based on a human sensory test to provide a final concentration that reproduces the smell, taste, or somatic sensation.

III. Program for determining a formulation of a combination consisting of multiple candidate substrates of smell, taste, or somatic sensation and reproducing a smell, taste, or somatic sensation of a target substance

The present invention relates to a program for determining a formulation of a combination consisting of multiple candidate substrates of smell, taste, or somatic sensation and reproducing a smell, taste, or somatic sensation of a target substance.

One illustrative embodiment of the present invention (hereinafter referred to as "the first illustrative embodiment" in this section) provides a program for determining a formulation of a combination consisting of multiple candidate substrates of smell, taste, or somatic sensation and reproducing a smell, taste, or somatic sensation of a target substance, the program causing a computer to implement the steps of:

(i) setting, with respect to one or multiple desired olfactory, taste, or somatosensory receptors, a function $f_iT$ representing a response intensity of the receptor for the target substance;
(ii) setting a function $f_iR$ representing a response intensity of the receptor for the combination consisting of the multiple candidate substrates of smell, taste, or somatic sensation and characterized by a concentration of each of the candidate substances; and
(iii) performing optimization for a functional F, and determining a concentration of each of the candidate substances when the functional F takes an optimal value as a concentration that reproduces the target smell, taste, or somatic sensation, wherein the functional F has as arguments all of error functions $g_i$ related to each receptor, and is minimized when the error functions are minimized for all of the receptors, and wherein an error function $g_i$ related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

The function $f_iT$ may be defined by formula (1) above. Therefore, step (i) is, or may include, the step of obtaining from memory the values of the parameters $\alpha_{i(T)}$ and $K_{i(T)}$ defining the function $f_iT$.

The function $f_iR$ may be defined by formula (2) above. Therefore, step (ii) is, or may include, the step of obtaining from memory the values of the parameters $\alpha_{ij}$ and $K_{ij}$ partially defining, i.e., characterizing, the function $f_iR$. It is noted that although formula (2) is also characterized by the concentration $C_j$ of each candidate substance as a parameter, this parameter is still unknown at step (ii).

The error function $g_i$ may be defined by formula (3) above, and the functional F may be defined by formula (5) above. Therefore, step (iii) can include the step of calculating the concentration $C_j$ of each candidate substance when the functional F takes an optimal value, when the functional F has as constants the obtained parameters $\alpha_{i(T)}$, $K_{i(T)}$, $\alpha_{ij}$, and $K_{ij}$, and has as a variable the concentration $C_j$ of each candidate substance. This step may include the step of changing the value of $C_j$ until the value of the functional F becomes the optimal value, using, for example, gradient methods such as the steepest descent method and Newton's method; evolutionary algorithms such as genetic algorithms; estimation algorithms based on probability distribution theory such as Bayesian optimization; and randomized algorithms such as Monte Carlo methods. A determination as to whether the functional F has become the optimal value may be made based on whether the change in the value of the functional F as the value of $C_j$ is changed according to a predetermined method is not more than or less than a predetermined threshold value. At that time, a constraint condition on concentration may be applied using a method such as the method of Lagrange multipliers.

One illustrative embodiment of the present invention (hereinafter referred to as "the second illustrative embodiment" in this section) provides a program for determining a formulation of a combination consisting of multiple candidate substrates of smell, taste, or somatic sensation and reproducing a smell, taste, or somatic sensation of a target substance, the program causing a computer to implement the step of:

determining a mixture ratio of the multiple candidate substrates of smell, taste, or somatic sensation such that with respect to one or multiple desired olfactory, taste, or somatosensory receptors, a dose response curve of the receptor for the target substance (hereinafter referred to as "the first dose response curve" in this section) and a dose response curve of the receptor for the combination consisting of the multiple candidate substrates of smell, taste, or somatic sensation (hereinafter referred to as "the second dose response curve" in this section) approximate each other, wherein the target substance is a single compound or a mixture of multiple compounds.

The first dose response curve may be defined by formula (1) above, and the second dose response curve may be defined by formula (2) above. Therefore, the step in the second illustrative embodiment may include steps (i) to (iii) in the first illustrative embodiment.

IV. System for determining a formulation of a combination consisting of multiple candidate substrates of smell, taste, or somatic sensation and reproducing a smell, taste, or somatic sensation of a target substance

The present invention relates to a system for determining a formulation of a combination consisting of multiple candidate substrates of smell, taste, or somatic sensation and reproducing a smell, taste, or somatic sensation of a target substance. Such a system may be implemented by cooperation between hardware resources constituting a computer and software such as the program as described above.

Therefore, one illustrative embodiment of the present invention provides a system for determining a formulation of a combination consisting of multiple candidate substrates of smell, taste, or somatic sensation and reproducing a smell, taste, or somatic sensation of a target substance, the system being configured to:

determine a mixture ratio of the multiple candidate substrates of smell, taste, or somatic sensation such that with respect to one or multiple desired olfactory, taste, or somatosensory receptors, a dose response curve of the receptor for the substance of interest and a dose response curve of the receptor for the combination consisting of the multiple candidate substrates of smell, taste, or somatic sensation approximate each other, wherein the substance of interest is a single compound or a mixture of multiple compounds.

Another illustrative embodiment of the present invention provides a system for producing a mixture that reproduces a smell, taste, or somatic sensation of a target substance, from a combination consisting of multiple candidate substrates of smell, taste, or somatic sensation, the system being configured to:

(i) set, with respect to one or multiple desired olfactory, taste, or somatosensory receptors, a function $f_iT$ representing a response intensity of the receptor for the substance of interest and having a concentration of the substance of interest as a variable;

(ii) set a function $f_iR$ representing a response intensity of the receptor for the combination consisting of the multiple candidate substrates of smell, taste, or somatic sensation and having a concentration of each of the candidate substances as a variable; and

(iii) perform optimization for a functional F, and determine a concentration of each of the candidate substances when the functional F takes an optimal value as a concentration of a recipe for the target smell, taste, or somatic sensation, wherein the functional F has as arguments all of error functions $g_i$ related to each receptor, and is minimized when the error functions are minimized for all of the receptors, and wherein an error function $g_i$ related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

V. Computer

One example of a hardware configuration of a computer that can be used to implement one illustrative embodiment of the present invention will be hereinafter described. The computer that can be used to implement one illustrative embodiment of the present invention may be any computer, for example, a personal computer, a tablet computer, a smart phone, or a computer on cloud.

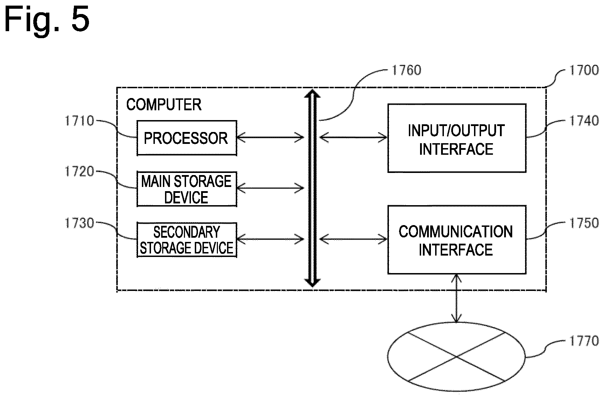

Fig. 5 shows one example of a hardware configuration of a computer. As shown in the figure, a computer 1700 mainly includes a processor 1710, a main storage device 1720, a secondary storage device 1730, an input/output interface 1740, and a communication interface 1750 as hardware resources, which are interconnected via bus lines 1760 including an address bus, a data bus, a control bus, and the like. An interface circuit (not shown) may be interposed between the bus lines 1760 and each hardware resource.

The processor 1710 performs overall control of the computer. It is noted that one computer may include multiple processors 1710. In this case, "processor" in the foregoing description may be the collective term for multiple processors 1710.

The main storage device 1720 provides a work area for the processor 1710 and is volatile memory, such as SRAM (Static Random Access Memory) or DRAM (Dynamic Random Access Memory).

The secondary storage device 1730 is non-volatile memory, such as an HDD, an SSD, or flash memory, that stores software such as a program, data, or the like. The program, data, or the like is loaded from the secondary storage device 1730 onto the main storage device 1720 via the bus lines 1760 at any time. Reference may be made to the secondary storage device 1730 as a computer-readable non-transitory storage medium. It is noted that the program includes instructions for causing the processor to execute desired processing.

The input/output interface 1740 performs either one or both of displaying information and receiving input of information, and includes a digital camera, a keyboard, a mouse, a display, a touch panel display, a microphone, a speaker, a temperature sensor, and the like.

The communication interface 1750 is connected to a network 1770, and transmits and receives data via the network 1770. The communication interface 1750 and the network 1770 can be wired or wirelessly connected. The communication interface 1750 may also obtain information related to the network, for example, information related to Wi-Fi access points and information related to base stations of a communication carrier.

It will be apparent to those skilled in the art that the computer 1700 can function as desired means, execute desired steps, and implement desired functions, through the cooperation of the hardware resources and software as exemplified above.

EXAMPLES

The present invention will be hereinafter described in detail based on examples; however, the present invention is not limited thereto. Based on the disclosure of the present specification, those skilled in the art can easily add modifications and alterations to the present invention, all of which are included within the technical scope of the present invention.

Example 1: Production of an olfactory receptor-related protein expressing cell line

In this example, a cell line expressing the olfactory receptor-related proteins hRTP1S, mRTP2, and $hG\alpha_{olf}$ was produced.

The mRNA sequence of human $G\alpha_{olf}$ is deposited in GenBank under accession number AF493893.1. The base sequence and the amino acid sequence of human $G\alpha_{olf}$ are listed as SEQ ID NOS: 1 and 2 in the Sequence Listing of the present application.

RTPs (receptor-transporting proteins) are a family of proteins known to have the function of promoting the functional expression of olfactory receptors. hRTP1S is one type of human RTP, which is deposited in GenBank: AY562235.1, and the base sequence and the amino acid sequence are listed as SEQ ID NOS: 3 and 4 in the Sequence Listing. mRTP2 is one type of mouse RTP, which is deposited in GenBank: AY562226.1, and the base sequence and the amino acid sequence are listed as SEQ ID NOS: 5 and 6 in the Sequence Listing.

The hRTP1S gene was located upstream and the mRTP2 gene was located downstream of the picornavirus-derived self-cleaving 2A peptide expressing gene (SEQ ID NO: 7), and this construct was inserted into the pIRESpuro3 vector (Takara Bio) to produce a vector coexpressing hRTP1S and mRTP2. Next, the $hG\alpha_{olf}$ gene was inserted into the pIRESneo3 vector (Takara Bio) to produce a vector expressing $hG\alpha_{olf}$. These two vectors were introduced into the human embryonic kidney cell line HEK293T (obtained from ECACC) using Lipofectamine 3000 (Thermo Fisher).

The resulting transformed HEK293T cells were cultured for 2 days at 37°C under 5% $CO_2$, and then the culturing was continued for additional 12 days using a selective medium containing 1 $\mu$g/mL puromycin (Thermo Fisher) and 200 $\mu$g/mL geneticin (Thermo Fisher) as the medium to produce a cell line (HEK-Olf) stably expressing hRTP1S, mRTP2, and $hG\alpha_{olf}$.

Example 2: Construction of an olfactory receptor response measurement system and response measurement

An olfactory receptor gene library (pFN21KSPc HaloTag-OR) prepared by inserting 351 human olfactory receptor genes obtained from a human cDNA library into the pFN21KSPc HaloTag vector was purchased from Kazusa Genome Technologies. Of the 351 types, olfactory receptors OR1A1, OR2B11, and the like were polymorphisms known to show low responses to known agonists, such that the olfactory receptors were modified into polymorphisms with high responsiveness for use in the subsequent test (Joel D. Mainland, et al., Nat Neurosci. 2014; 17(1): p. 114-120).

The cell line HEK-Olf obtained in Example 1 was prepared in DMEM medium (Thermo Fisher) and then seeded in a 96-well plate (Corning) at $1 \times 10^4$ cells/well. Subsequently, pFN21KSPc HaloTag-OR and pGL4.29[luc2P/CRE/Hygro] Vector (Promega) were introduced into the HEK-Olf with ViaFect (trademark) Transfection Reagent (Promega). At this time, pFN21KSPc HaloTag-OR and pGL4.29[luc2P/CRE/Hygro] Vector were prepared to give 70 ng/well and 30 ng/well, respectively. Using each olfactory receptor expressing HEK-Olf thus constructed, measurement of responses of the olfactory receptors was performed as follows.

Each olfactory receptor expressing HEK-Olf obtained above was cultured for 24 hours at 37°C under 5% $CO_2$; then the medium was removed, and test compounds (candidate substances) prepared appropriately in DMEM medium were added in 50 $\mu$L aliquots. DMEM medium containing no test compound was added to negative control wells, and forskolin adjusted to 3 $\mu$M was added to positive control wells. After 3 hours of reaction at 37°C under 5% $CO_2$, responses of the olfactory receptors were obtained by luminescence measurement using ONE-Glo (trademark) Luciferase Assay kit (Promega).

The response intensity R of an olfactory receptor at each concentration was calculated using the luminescence intensity (RLU) obtained in the measurement, based on formula (6) below:

[Formula 7]

$$R = \frac{RLU_{Sample} - RLU_{negative\ control}}{RLU_{Positive\ control} - RLU_{negative\ control}} \times 100 \quad (6)$$

Furthermore, using the curve fitting software XLFit (IDES), the maximum response intensity and EC50 of the dose response curve were derived.

Based on the above-described procedure, 351 human olfactory receptors were measured for responses to coumarin as a fragrance. As a result, six olfactory receptors were confirmed to show dose-dependent responses to coumarin. All of these six olfactory receptors were classified as class II.

Example 3: Reproduction of a dose response curve of the target substance (fragrance)

The present inventors have conceived that if the dose response curve of a target fragrance for an olfactory receptor can be reproduced by the dose response curve of a mixture of other fragrances, then the smell of the fragrance of interest can be reproduced by using the mixture.

Agonists for olfactory receptors are hereinafter considered as candidate substances. In general, the response intensity (R) at the concentration (C) of an agonist is given by formula (7) using the maximum response intensity ($\alpha$) and EC50 (K). Moreover, it is known that the response intensity $R_{mix}$ of a mixture obtained by mixing n types of agonists for an identical receptor at concentrations $C_1$, $C_2$ .... $C_n$ is estimated by formula (8) using the EC50 ($K_1$, $K_2$ .... $K_n$) and the maximum response intensity ($\alpha_1$, $\alpha_2$ .... $\alpha_n$) of each agonist (Howard GJ et al., J. Theor. Biol. 2009 Aug 7; 259(3): 469-477).

[Formula8]

$$R = \frac{\alpha C}{K + C} \qquad (7)$$

$$R_{mix} = \frac{\frac{\alpha_1 C_1}{K_1} + \frac{\alpha_2 C_2}{K_2} + \cdots + \frac{\alpha_n C_n}{K_n}}{1 + \frac{C_1}{K_1} + \frac{C_2}{K_2} + \cdots + \frac{C_n}{K_n}} \qquad (8)$$

Here, when the mixture obtained by mixing the agonists at concentrations $C_1$, $C_2$ ... $C_n$ is used as a stock solution and diluted uniformly at dilution ratio X while maintaining the concentration ratio, the concentrations of the agonists in the diluted solution are $XC_1$, $XC_2$ ... $XC_n$. By substituting these into the respective concentrations in formula (8), the response intensity ($R_{(eff)}$) at dilution ratio X of the solution obtained by mixing the n types of compounds at concentrations $C_1$, $C_2$ ... $C_n$ in the stock solution can be derived (formula (9)). Moreover, the effective EC50 ($K_{(eff)}$) and maximum response intensity ($\alpha_{(eff)}$) of a dose response curve having the dilution ratio as a variable can be obtained using formulae (10) and (11) from the values of the concentration of each agonist in the stock solution, the EC50 of each agonist, and the maximum response intensity of each agonist.
[Formula 9]

$$
R_{(eff)} = \frac{\frac{\alpha_1 XC_1}{K_1} + \frac{\alpha_2 XC_2}{K_2} + \cdots + \frac{\alpha_n XC_n}{K_n}}{1 + \frac{XC_1}{K_1} + \frac{XC_2}{K_2} + \cdots + \frac{XC_n}{K_n}}
$$

$$
= \frac{\frac{\left(\frac{\alpha_1 C_1}{K_1} + \frac{\alpha_2 C_2}{K_2} + \cdots + \frac{\alpha_n C_n}{K_n}\right)}{\frac{C_1}{K_1} + \frac{C_2}{K_2} + \cdots + \frac{C_n}{K_n}} X}{\frac{1}{\frac{C_1}{K_1} + \frac{C_2}{K_2} + \cdots + \frac{C_n}{K_n}} + X}
$$

$$
= \frac{\alpha_{(eff)} X}{K_{(eff)} + X} \qquad (9)
$$

$$
\alpha_{(eff)} = \frac{\left(\frac{\alpha_1 C_1}{K_1} + \frac{\alpha_2 C_2}{K_2} + \cdots + \frac{\alpha_n C_n}{K_n}\right)}{\frac{C_1}{K_1} + \frac{C_2}{K_2} + \cdots + \frac{C_n}{K_n}} \qquad (10)
$$

$$
K_{(eff)} = \frac{1}{\frac{C_1}{K_1} + \frac{C_2}{K_2} + \cdots + \frac{C_n}{K_n}} \qquad (11)
$$

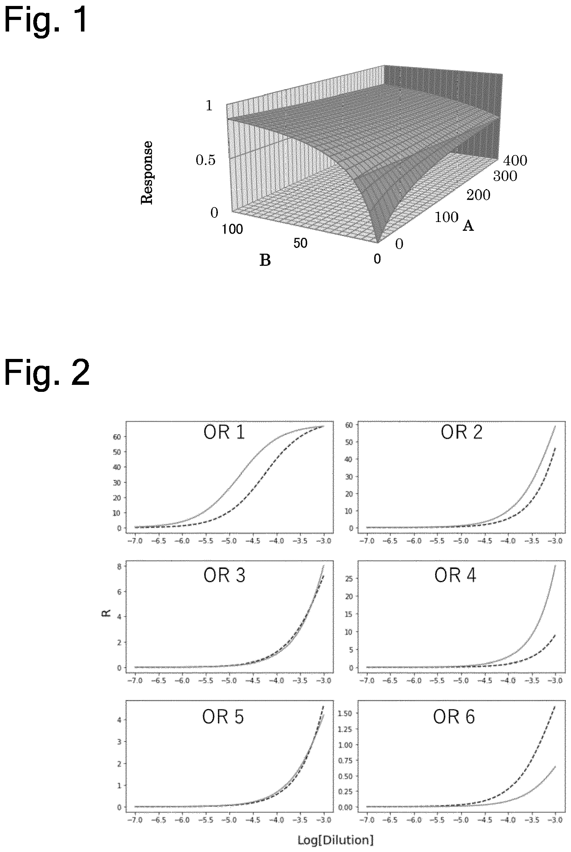

Fig. 1 shows a dose response curve model obtained using formula (9), for a mixture of agonists A and B with different EC50s and maximum response intensities. As shown in Fig. 1, the EC50 and the maximum response intensity of the mixture can be manipulated by varying the mixture ratio of agonists A and B.

When the agonists forming the stock solution contain a mixture, the formula above holds even if the dilution ratio relative to any specified concentration taken as 1 is employed as the concentration of the mixture. That is, when the concentration of an undiluted stock solution of the mixture, i.e., the concentration at a dilution ratio of 1, is hypothetically taken as the concentration corresponding to 1 mol/L of a single compound, the concentration can be expressed on a scale equivalent to the molar concentration in a simulated manner.

To reproduce the dose response curve of the fragrance of interest by using a mixture of other fragrances, the concentration of each fragrance in the stock solution may be obtained such that the dose response curve obtained according to formula (9) approximates the dose response curve of the fragrance of interest. Moreover, to avoid the mixture from exhibiting a smell that is not possessed by the fragrance of interest, it is desirable that the dose response curve also approximate that for a receptor for which the fragrance of interest is not a ligand. That is, it is desirable to adjust the dose response curve of the mixture such that the response to the receptor approaches zero at least in the range of concentrations where actual use is contemplated. Specific examples of methods include reducing the value of $\alpha_{a(eff)}$; and increasing the value of $K_{a(eff)}$ sufficiently. Here, an error function $g_a$ between the dose response curve of the fragrance of interest and the dose response curve of the mixture for olfactory receptor a is defined using the curve

parameters K and $\alpha$, as follows:
[Formula 10]

$$g_a[\kappa_{a(T)}, \alpha_{a(T)}, \kappa_{a(eff)}, \alpha_{a(eff)}] := $$

$$\begin{cases} \left(\dfrac{\kappa_{a(eff)} - \kappa_{a(T)}}{\overline{\kappa_{(T)}}}\right)^2 + \left(\dfrac{\alpha_{a(eff)} - \alpha_{a(T)}}{\overline{\alpha_{(T)}}}\right)^2 & for \ a \ s.t. \ \alpha_{i(T)} > 0 \\ \left(\dfrac{\alpha_{a(eff)}}{\overline{\alpha_{(T)}}}\right)^2 & for \ a \ s.t. \ \alpha_{i(T)} = 0 \end{cases} \qquad (12)$$

wherein the subscript a represents the type of olfactory receptor; T represents the fragrance of interest; and eff represents the mixture of fragrances. $\kappa$ is the common logarithm value of EC50.

[Formula 11]

$$\kappa_{a(eff)} := log[K_{a(eff)}], \kappa_{a(T)} := log[K_{a(T)}]$$

Furthermore, since the typical values of the curve parameters are different, normalization was performed based on the amounts defined as follows:

[Formula 12]

$$\overline{\kappa_{(T)}} := \frac{1}{N} \sum_{a, \alpha_{a(T)} \neq 0} \kappa_{a(T)}, \overline{\alpha_{(T)}} := \frac{1}{N} \sum_{a, \alpha_{a(T)} \neq 0} \alpha_{a(T)}$$

wherein N is the number of receptors for which the fragrance of interest is a ligand. Therefore:

[Formula 13]

$$\overline{\kappa_{(T)}}, \overline{\alpha_{(T)}}$$

is the average value of each parameter for the olfactory receptors that respond to the fragrance of interest.

Subsequently, to approximate the multiple receptors that respond to the fragrance of interest, with respect to all of the olfactory receptors of interest, the functional F as an indicator characterizing the difference between the dose response curve of the fragrance of interest and the dose response curve of the fragrance mixture was obtained according to formula (13), and the concentration of the constituent components in the stock solution at which the value of F was minimized was found.

[Formula 14]

$$F := \sum_a g_a[K_{a(T)}, \alpha_{a(T)}, K_{a(eff)}, \alpha_{a(eff)}] \qquad (13)$$

In view of the foregoing considerations, an attempt was made to reproduce the dose response curves for coumarin of the six olfactory receptors OR1, OR2, OR3, OR4, OR5, and OR6, which were confirmed to respond to coumarin in Example 2, by using a mixture of other fragrances.

A search for fragrances to which the six olfactory receptors respond resulted in the identification of piperonal, $\gamma$-heptalactone, and menthone. Of the six olfactory receptors, the olfactory receptor(s) that respond to piperonal, $\gamma$-heptalactone, and menthone are as shown in the table below.

[Table 2]

| Olfactory Receptor | Piperonal | $\gamma$-Heptalactone | Menthone |
|---|---|---|---|
| OR 1 | ○ | ○ | |

(continued)

| Olfactory Receptor | Piperonal | γ-Heptalactone | Menthone |
|:---:|:---:|:---:|:---:|
| OR 2 | ○ | | |
| OR 3 | | ○ | ○ |
| OR 4 | ○ | | |
| OR 5 | ○ | ○ | |
| OR 6 | ○ | | |

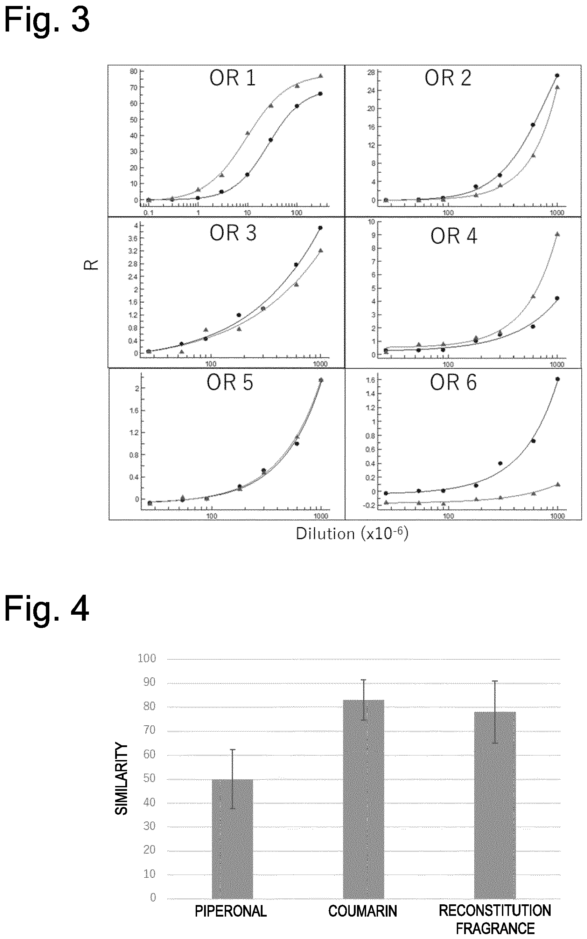

Subsequently, the maximum response intensities and the EC50s of coumarin, piperonal, γ-heptalactone, and menthone for each olfactory receptor were applied to formula (13), and then optimization calculations were performed using a numerical gradient method, and using a computer program created by using the scipy package in python language. The results showed that the value of F was minimized when piperonal, γ-heptalactone, and menthone were mixed at concentrations in the stock solution of 0.6 M, 0.39 M, and 0.01 M, respectively. A predicted dose response curve of the mixture for each receptor is as shown in Fig. 2. Based on the calculation results as described above, responses of the mixture of piperonal, γ-heptalactone, and menthone respectively at 0.6 M, 0.39 M, and 0.01 M for the six olfactory receptors were measured and, as a result, dose response curves approximating those of coumarin equivalent to the dose response curves as predicted in Fig. 2 were obtained (Fig. 3). Thus, this mixture was designated as a fragrance reproducing the smell of coumarin (coumarin reconstitution fragrance).

Example 4: Confirmation of similarity between the fragrance of interest and the reconstitution fragrance by sensory evaluation

In this example, similarity in odor quality between coumarin and the coumarin reconstitution fragrance obtained in Example 3 was verified by sensory evaluation. In the sensory evaluation, the verification was performed by a panel of five trained experts. The similarity in odor was obtained by evaluating the similarity between the headspace aromas of coumarin presented as a reference and each evaluation sample presented blindly, using a Visual Analog Scale (VAS). The evaluation samples used herein were the coumarin reconstitution fragrance of Example 3 and additionally, coumarin, and piperonal, which, of the constituent components of the coumarin reconstitution fragrance, exhibits olfactory receptor responses closest to those of coumarin. Each fragrance used for the evaluation was prepared with propylene glycol at the concentration shown in the table below.

[Table 3]

| Sample | Concentration (M) | | | |
| :---: | :---: | :---: | :---: | :---: |
| | Coumarin | Piperonal | γ-Heptalactone | Menthone |
| Coumarin | 0.1 | - | - | - |
| Coumarin Reconstitution Fragrance | - | 0.06 | 0.039 | 0.001 |
| Piperonal | - | 0.06 | - | - |

The results are shown in Fig. 4. The results of the sensory evaluation confirmed that the coumarin reconstitution fragrance exhibits very high similarity comparable to coumarin presented as an evaluation sample. In contrast, piperonol alone exhibits low similarity, which suggests that it is important to have olfactory receptor responses approximate to those of the fragrance of the target substance, in order to improve the similarity in odor quality.

Example 5: Reproduction of a dose response curve of the target substance (fragrance)

Using the same methods as in Examples 2 and 3, an attempt was made to measure olfactory receptor responses of lavender oil and reproduce the responses by using a mixture of other fragrances.

Table 4: Responses of Lavender Oil and Fragrances

[Table 4]

|  | Lavender Oil | o-Hydroxycinnamic Acid | Neroli Oil | Nootkatone | Ethyl Valerate |
|---|---|---|---|---|---|
| OR7 | ○ | ○ |  | ○ |  |
| OR3 | ○ |  | ○ |  |  |
| OR8 | ○ | ○ |  | ○ |  |
| OR9 | ○ | ○ | ○ | ○ | ○ |
| OR10 |  |  |  | ○ |  |

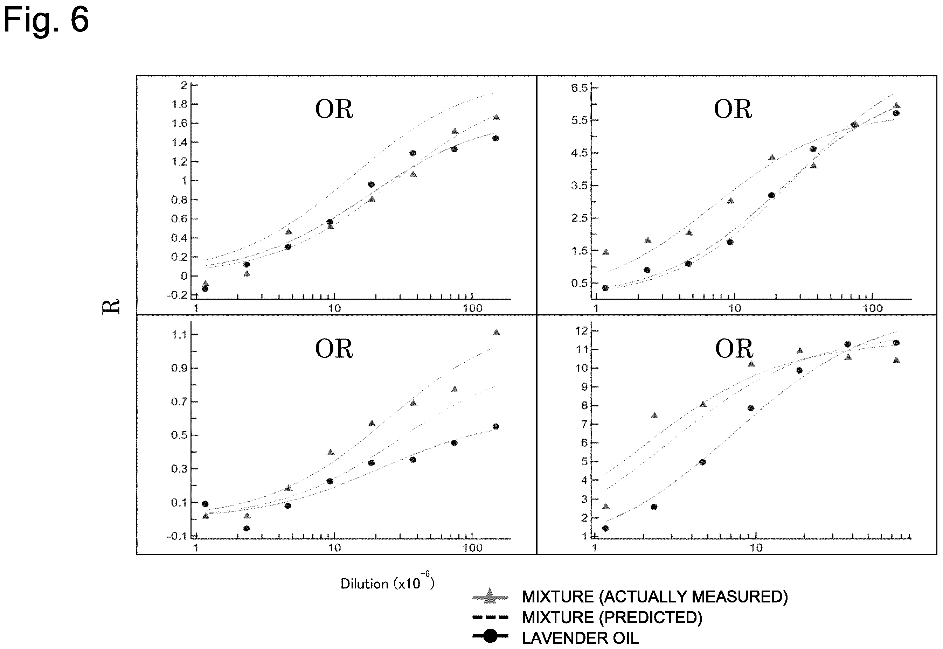

Four olfactory receptors (OR7, OR3, OR8, and OR9) were confirmed to respond to lavender oil (Table 4). A search for fragrances to which the four olfactory receptors respond resulted in the identification of o-hydroxycinnamic acid, neroli oil, nootkatone, and ethyl valerate. Of the four olfactory receptors, the olfactory receptor(s) that respond to o-hydroxycinnamic acid, neroli oil, nootkatone, and ethyl valerate are as shown in Table 4. The maximum response intensity and the EC50 of each fragrance were applied, and then optimization calculations were performed to give a similar response to that of lavender oil. The results showed that the value of F was minimized when o-hydroxycinnamic acid, neroli oil, nootkatone, and ethyl valerate were mixed at concentrations in the stock solution of 1.09 M, 1.23 x dilution, 1.04 M, and 1.16 M, respectively. Based on these calculation results, responses of the mixture of o-hydroxycinnamic acid, neroli oil, nootkatone, and ethyl valerate respectively at concentrations in the stock solution of 1.09 M, 1.23 x dilution, 1.04 M, and 1.16 M for the four olfactory receptors were measured and, as a result, dose response curves approximating those of lavender oil were obtained (Fig. 6). Thus, this mixture was designated as a fragrance reproducing the smell of lavender oil (lavender oil reconstitution fragrance).

Subsequently, similarities were compared between the lavender oil and the lavender reconstitution oil fragrance by sensory evaluation as in Example 4. Specifically, in the sensory evaluation, the verification was performed by a panel of five trained experts. The similarity in odor was obtained by evaluating the similarity between the headspace aromas of lavender oil presented as a reference and each evaluation sample presented blindly, using a Visual Analog Scale (VAS). The evaluation samples were prepared with propylene glycol at the concentrations (formulation ratios) shown in the table below.

[Table 5]

| Sample | Concentration ($\times 10^{-3}$ M or $\times 10^{-3}$ Dilution) | | | | |
|---|---|---|---|---|---|
|  | Lavender Oil | o-Hydroxycinnamic Acid | Neroli Oil | Nootkatone | Ethyl Valerate |
| Lavender Oil | 50 | - | - | - |  |
| Lavender Oil Reconstitution Fragrance | - | 54 | 61 | 52 | 58 |

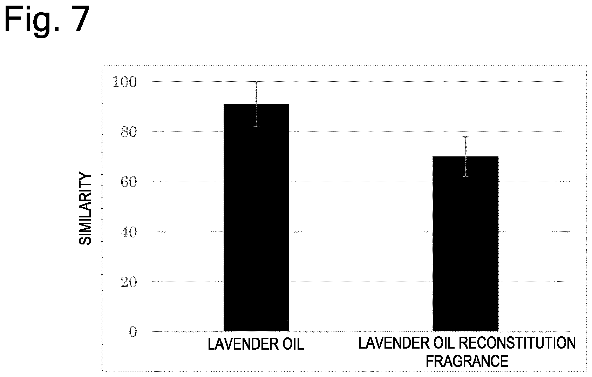

The sensory evaluation revealed that the lavender oil reconstitution fragrance exhibits high similarity, although slightly inferior to that of the lavender oil presented as an evaluation sample (Fig. 7).

INDUSTRIAL APPLICABILITY

The present invention makes it possible to determine a suitable formulation of a combination consisting of multiple candidate substrates of smell, taste, or somatic sensation and reproducing a smell, taste, or somatic sensation of a target substance. The methods, programs, and the like of the present invention allow the smell, taste, or somatic sensation of the target substance to be reproduced by using the suitable formulation of the combination consisting of the multiple candidate substrates of smell, taste, or somatic sensation, even in circumstances where the target substance is difficult to handle, difficult to obtain, or expensive.

**Claims**

1. A method for determining a formulation of a combination consisting of multiple candidate substrates of smell, taste, or somatic sensation and reproducing a smell, taste, or somatic sensation of a target substance, the method comprising:

determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other, wherein the target substance is a single compound or a mixture of multiple compounds.

2. A method for determining a formulation of a combination consisting of multiple candidate substrates of smell, taste, or somatic sensation and reproducing a smell, taste, or somatic sensation of a target substance, the method comprising:

(i) step 1 of setting, with respect to one or multiple desired olfactory, taste, or somatosensory receptors, a function $f_iT$ representing a response intensity of the receptor for the target substance;
(ii) step 2 of setting a function $f_iR$ representing a response intensity of the receptor for the combination consisting of the multiple candidate substrates of smell, taste, or somatic sensation and **characterized by** a concentration of each of the candidate substances;
(iii) step 3 of setting an error function g characterizing an error between the function $f_iT$ and the function $f_iR$ for each receptor, wherein the error function is minimized when the function $f_iT$ and the function $f_iR$ match each other;
(iv) step 4 of setting a functional F having as arguments all of error functions $g_i$ related to each receptor obtained in step 3, wherein the functional F is minimized when the error functions are minimized for all of the receptors; and
(v) step 5 of performing optimization for the functional F, and determining a concentration of each of the candidate substances when the functional F takes an optimal value as a concentration that reproduces the target smell, taste, or somatic sensation,

wherein the target substance is a single compound or a mixture of multiple compounds.

3. The method according to claim 1 or 2, wherein the one or multiple olfactory, taste, or somatosensory receptors include a receptor for which the target substance is a ligand.

4. The method according to claim 1 or 2, wherein the one or multiple olfactory, taste, or somatosensory receptors include all of receptors for which the target substance is a ligand.

5. The method according to any one of claims 1 to 4, wherein the one or multiple olfactory, taste, or somatosensory receptors include a receptor for which at least one of the smell, taste, or somatosensory candidate substances is a ligand.

6. The method according to any one of claims 1 to 5, wherein at least one of the multiple candidate substrates of smell, taste, or somatic sensation is a ligand for a receptor for which the target substance is a ligand.

7. The method according to any one of claims 1 to 6, wherein the receptors are multiple olfactory, taste, or somatosensory receptors.

8. The method according to any one of claims 2 to 7, wherein the function $f_iT$ includes as parameters a maximum response intensity $\alpha$ and an EC50 (a concentration representing 50% of a maximum response from a minimal value) of the target substance for the one or multiple olfactory, taste, or somatosensory receptors of interest.

9. The method according to any one of claims 2 to 8, wherein the function $f_iR$ includes as parameters a maximum response intensity $\alpha$ and an EC50 of the combination of the multiple candidate substances for the one or multiple olfactory, taste, or somatosensory receptors of interest.

10. The method according to any one of claims 2 to 9, further comprising:
(vi) step 6 of adjusting the concentration that reproduces the smell, taste, or somatic sensation obtained in step 5 based on a human sensory test to provide a final concentration that reproduces the smell, taste, or somatic sensation.

11. A method for producing a mixture that reproduces a smell, taste, or somatic sensation of a target substance, from a combination consisting of multiple candidate substrates of smell, taste, or somatic sensation, the method comprising:

(i) step 1 of setting, with respect to one or multiple desired olfactory, taste, or somatosensory receptors, a

function $f_iT$ representing a response intensity of the receptor for the target substance;

(ii) step 2 of setting a function $f_iR$ representing a response intensity of the receptor for the combination of the multiple candidate substrates of smell, taste, or somatic sensation and **characterized by** a concentration of each of the candidate substances;

(iii) step 3 of setting an error function g characterizing an error between the function $f_iT$ and the function $f_iR$ for each receptor, wherein the error function is minimized when the function $f_iT$ and the function $f_iR$ match each other;

(iv) step 4 of setting a functional F having as arguments all of error functions g; related to each receptor obtained in step 3, wherein the functional F is minimized when the error functions are minimized for all of the receptors;

(v) step 5 of performing optimization for the functional F, and determining a concentration of each of the candidate substances when the functional F takes an optimal value as a concentration that reproduces the target smell, taste, or somatic sensation, and

(vi) step 6 of mixing the candidate substances at the concentrations determined in step 5 to produce a mixture, wherein the target substance is a single compound or a mixture of multiple compounds.

12. A program for determining a formulation of a combination consisting of multiple candidate substrates of smell, taste, or somatic sensation and reproducing a smell, taste, or somatic sensation of a target substance, the program causing a computer to implement the step of:

determining a mixture ratio of the multiple candidate substrates of smell, taste, or somatic sensation such that with respect to one or multiple desired olfactory, taste, or somatosensory receptors, a dose response curve of the receptor for the target substance and a dose response curve of the receptor for the combination consisting of the multiple candidate substrates of smell, taste, or somatic sensation approximate each other, wherein the target substance is a single compound or a mixture of multiple compounds.

13. A program for determining a formulation of a combination consisting of multiple candidate substrates of smell, taste, or somatic sensation and reproducing a smell, taste, or somatic sensation of a target substance, the program causing a computer to implement the steps of:

(i) setting, with respect to one or multiple desired olfactory, taste, or somatosensory receptors, a function $f_iT$ representing a response intensity of the receptor for the target substance;

(ii) setting a function $f_iR$ representing a response intensity of the receptor for the combination consisting of the multiple candidate substrates of smell, taste, or somatic sensation and **characterized by** a concentration of each of the candidate substances; and

(iii) performing optimization for a functional F, and determining a concentration of each of the candidate substances when the functional F takes an optimal value as a concentration that reproduces the target smell, taste, or somatic sensation, wherein the functional F has as arguments all of error functions g; related to each receptor, and is minimized when the error functions are minimized for all of the receptors, and wherein an error function g; related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

14. A system for determining a formulation of a combination consisting of multiple candidate substrates of smell, taste, or somatic sensation and reproducing a smell, taste, or somatic sensation of a target substance, the system being configured to:

determine a mixture ratio of the multiple candidate substrates of smell, taste, or somatic sensation such that with respect to one or multiple desired olfactory, taste, or somatosensory receptors, a dose response curve of the receptor for the substance of interest and a dose response curve of the receptor for the combination consisting of the multiple candidate substrates of smell, taste, or somatic sensation approximate each other, wherein the substance of interest is a single compound or a mixture of multiple compounds.

15. A system for producing a mixture that reproduces a smell, taste, or somatic sensation of a target substance, from a combination consisting of multiple candidate substrates of smell, taste, or somatic sensation, the system being configured to:

(i) set, with respect to one or multiple desired olfactory, taste, or somatosensory receptors, a function $f_iT$ representing a response intensity of the receptor for the substance of interest;

(ii) set a function $f_iR$ representing a response intensity of the receptor for the combination consisting of the multiple candidate substrates of smell, taste, or somatic sensation and having a concentration of each of the candidate substances as a variable; and

(iii) perform optimization for a functional F, and determine a concentration of each of the candidate substances when the functional F takes an optimal value as a concentration of a recipe for the target smell, taste, or somatic sensation, wherein the functional F has as arguments all of error functions $g_i$ related to each receptor, and is minimized when the error functions are minimized for all of the receptors, and wherein an error function $g_i$ related to each receptor characterizes an error between the function $f_iT$ and the function $f_iR$ for each receptor, and is minimized when the function $f_iT$ and the function $f_iR$ match each other,

wherein the target substance is a single compound or a mixture of multiple compounds.

Fig. 1

Fig. 2

Fig. 3

Dilution (x10⁻⁶)

Fig. 4

# Fig. 5

1760

1700

COMPUTER

1710 — PROCESSOR

1720 — MAIN STORAGE DEVICE

1730 — SECONDARY STORAGE DEVICE

1740 — INPUT/OUTPUT INTERFACE

1750 — COMMUNICATION INTERFACE

1770

# Fig. 6

# Fig. 7

## INTERNATIONAL SEARCH REPORT

| | International application No. |
|---|---|
| | PCT/JP2021/019359 |

**A. CLASSIFICATION OF SUBJECT MATTER**
C12Q 1/02(2006.01)i; C11B 9/00(2006.01)i; G01N 21/76(2006.01)i
FI: C12Q1/02 ZNA; C11B9/00 P; C11B9/00 X; C11B9/00 N; G01N21/76
According to International Patent Classification (IPC) or to both national classification and IPC

**B. FIELDS SEARCHED**

Minimum documentation searched (classification system followed by classification symbols)
C12Q1/00-3/00; C11B1/00-15/00; C11C1/00-5/02; G01N21/75-21/83; G01N33/00-33/46; G01N33/48-33/98

Documentation searched other than minimum documentation to the extent that such documents are included in the fields searched
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| A | JP 2016-530610 A (COMMONWEALTH SCIENTIFIC AND INDUSTRIAL RESEARCH ORGANISATION) 29 September 2016 (2016-09-29) claims, paragraphs [0040]-[0107] | 1-15 |
| A | JP 2003-279459 A (RIKOGAKU SHINKOKAI) 02 October 2003 (2003-10-02) claims, paragraphs [0011]-[0012], [0031]-[0069] | 1-15 |
| A | JP 2005-43072 A (TOKYO INSTITUTE OF TECHNOLOGY) 17 February 2005 (2005-02-17) claims, paragraphs [0004]-[0009], [0011]-[0035] | 1-15 |
| A | JP 2018-109099 A (KAO CORP.) 12 July 2018 (2018-07-12) claims, paragraphs [0014]-[0020], [0027]-[0100] | 1-15 |

☒ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 July 2021 (05.07.2021) | 13 July 2021 (13.07.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2021/019359

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2008-308649 A (KYUSHU UNIVERSITY) 25 December 2008 (2008-12-25) claims, paragraphs [0007]-[0010], [0014]-[0070] | 1-15 |
| A | WO 2018/190118 A1 (TAKASAGO INTERNATIONAL CORPORATION) 18 October 2018 (2018-10-18) claims, examples | 1-15 |

Form PCT/ISA/210 (continuation of second sheet) (January 2015)

**INTERNATIONAL SEARCH REPORT**
Information on patent family members

International application No.

PCT/JP2021/019359

| Patent Documents referred in the Report | Publication Date | Patent Family | Publication Date |
|---|---|---|---|
| JP 2016-530610 A | 29 Sep. 2016 | WO 2015/006812 A1 claims, page 7, line 25 to page 19, line 32 | |
| JP 2003-279459 A | 02 Oct. 2003 | (Family: none) | |
| JP 2005-43072 A | 17 Feb. 2005 | (Family: none) | |
| JP 2018-109099 A | 12 Jul. 2018 | (Family: none) | |
| JP 2008-308649 A | 25 Dec. 2008 | (Family: none) | |
| WO 2018/190118 A1 | 18 Oct. 2018 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003279459 A
- JP 2005043072 A
- JP 2008308649 A
- JP 2018109099 A
- WO 2018190118 A

**Non-patent literature cited in the description**

- **JOEL D. MAINLAND et al.** *Nat Neurosci.,* 2014, vol. 17 (1), 114-120
- **HOWARD GJ et al.** *J. Theor. Biol.,* 07 August 2009, vol. 259 (3), 469-477
- **SAITO H et al.** *Sci Signal,* 2009, vol. 2 (60), 9
- **JOEL D. MAINLAND et al.** *Scientific Data,* 2015, vol. 2, 150002
- **AMRITA SAMANTA et al.** *Subcell Biochem.,* 2018, vol. 87, 141-165
- Kagaku Juyouno Kagaku: Nioi Aji Feromon Bunshi Kara Koudou Made. Chemosensory sciences: odor, taste and pheromones, from molecules to behaviors. Kagaku-Dojin, 2012